(19) Europäisches Patentamt
European Patent Office
Office européen des brevets

(11) Veröffentlichungsnummer: **0 012 822**
**B1**

# (12) EUROPÄISCHE PATENTSCHRIFT

(45) Veröffentlichungstag der Patentschrift: **01.12.82**

(51) Int. Cl.³: **C 07 D 233/50**, A 61 K 31/415

(21) Anmeldenummer: **79104381.3**

(22) Anmeldetag: **08.11.79**

## (54) Neue 3,4-disubstituierte 2-Phenylimino-imidazolidine, deren Säureadditionssalze, diese enthaltende Arzneimittel und Verfahren zu deren Herstellung.

(30) Priorität: **18.12.78 DE 2854659**

(43) Veröffentlichungstag der Anmeldung: **09.07.80 Patentblatt 80/14**

(45) Bekanntmachung des Hinweises auf die Patenterteilung: **01.12.82 Patentblatt 82/48**

(84) Benannte Vertragsstaaten: **AT BE CH DE FR GB IT LU NL SE**

(56) Entgegenhaltungen:
**DE-A-1 445 538**
**DE-A-1 545 628**
**DE-A-2 806 775**
**FR-M- 6 998**
**NL-A-6 800 852**

**Die Akte enthält technische Angaben, die nach dem Eingang der Anmeldung eingereicht wurden und die nicht in dieser Patentschrift enthalten sind.**

(73) Patentinhaber: **C.H. BOEHRINGER SOHN, Postfach 200, D-6507 Ingelheim am Rhein (DE)**

(72) Erfinder: **Stähle, Helmut, Dr., Rotweinstrasse 23, D-6507 Ingelheim/Rhein (DE)**
Erfinder: **Köppe, Herbert, Dr., Neuweg 72, D-6507 Ingelheim/Rhein (DE)**
Erfinder: **Kummer, Werner, Dr., Georg-Scheuing-Strasse 15, D-6507 Ingelheim/Rhein (DE)**
Erfinder: **Walland, Alexander, Dr., Wilhelm-Leuschner-Strasse 20, D-6507 Ingelheim/Rhein (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

# Neue 3,4-disubstituierte 2-Phenylimino-imidazolidine, deren Säureadditionssalze, diese enthaltende Arzneimittel und Verfahren zu deren Herstellung

2-Phenylimino-imidazolidine beanspruchen wegen ihrer hervorragenden pharmakologischen und therapeutischen Eigenschaften seit langem ein starkes Interesse. Verbindungen dieses Typs sind daher in der Literatur vielfach beschrieben worden und z.B. in den belgischen Patentschriften Nr. 623 305, 653 933, 687 656, 687 657 und 705 944 offenbart. In diesen Schrifttumstellen sind auch die wesentlichen Verfahren zur Herstellung von 2-Phenylimino-imidazolidinen angegeben. Bisher standen die 2,6-disubstituierten Phenylimino-imidazolidine und deren blutdrucksenkende Eigenschaften sowie deren Wirkung auf das Zentralnervensystem im Vordergrund des Interesses.

Es wurde nun gefunden, dass eine Reihe 3,4-disubstituierter 2-Phenylimino-imidazolidine praktisch keine zentrale, sondern vorwiegend eine periphere praesynaptische α-adrenerge Stimulierung bewirken, wobei der Blutdruck nicht beeinflusst wird.

Gegenstand der Erfindung sind neue, in 3- und 4-Stellung disubstituierte 2-Phenylimino-imidazolidine der allgemeinen Formel (I)

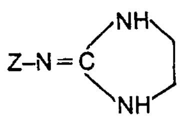
(I)

sowie deren physiologisch verträgliche Säureadditionssalze mit wertvollen therapeutischen Eigenschaften. In der Formel (I) bedeutet Z einen Rest aus der Gruppe 3-Fluor-4-methylphenyl, 4-Fluor-3-methylphenyl, 4-Fluor-3-nitrophenyl, 4-Fluor-3-chlorphenyl, 4-Fluor-3-aminophenyl.

Die Herstellung der neuen Verbindungen der Formel (I) kann nach einem der folgenden Verfahren erfolgen:

a) Umsetzung einer Verbindung der allgemeinen Formel (II)

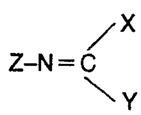
(II)

in der Z die oben angegebene Bedeutung besitzt und X und Y, die gleich oder verschieden sein können, ein Chloratom, eine Alkylthiogruppe mit 1 bis 4 Kohlenstoffatomen, eine Amino- oder Ammoniumgruppe bedeuten, mit Äthylendiamin bzw. dessen Säureadditionssalzen.

Ausgangsverbindungen der Formel (II) sind z.B. Isocyaniddichloride, S-Alkylthioharnstoffe bzw. deren Säureadditionssalze, wobei die Alkylreste 1 bis 4 Kohlenstoffatome enthalten oder Guanidine, auch in Form von Säureadditionssalzen.

Die Umsetzung erfolgt bei Temperaturen zwischen 0 und 200 °C in Abhängigkeit von den Resten X und Y. Als Lösungsmittel können polare protische, polare aprotische oder unpolare verwendet werden. In Abhängigkeit von den Resten X und Y kann die Umsetzung aber auch ohne Anwendung von Lösungsmitteln bei erhöhter Temperatur erfolgen. Bedeutet einer oder beide Reste X und Y ein Chloratom, so empfiehlt es sich, ein säurebindendes Mittel bei der Reaktion zu verwenden. Die Reaktionszeit richtet sich nach der Reaktivität der eingesetzten Komponenten und schwankt zwischen einigen Minuten und mehreren Stunden.

b) Hydrolytische Abspaltung eines aliphatischen Acylrestes aus einem 1-Acyl-2-phenylamino-imidazolin der allgemeinen Formel (III)

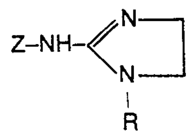
(III)

in der Z wie oben angegeben definiert ist und R einen aliphatischen Acylrest bedeutet. Die hydrolytische Abspaltung der Acylgruppe erfolgt am besten mittels niederer Alkohole, z.B. Methanol oder verdünnten Mineralsäuren, in denen die 1-Acyl-Verbindungen unter Rückfluss gekocht werden.

Die als Ausgangsmaterial benötigten 1-Acyl-2-phenyl-aminoimidazoline werden durch Umsetzung eines Anilins der allgemeinen Formel (IV)

$$Z\text{–}NH_2 \qquad (IV)$$

worin Z die obige Bedeutung besitzt, mit 1-Acyl-imidazolidin-2-on in Gegenwart von Phosphoroxychlorid nach dem in der DE-A-2 316 377 angegebenen Verfahren hergestellt.

c) Reduktion von 2-(4-Fluor-3-nitrophenyl-imino)-imidazolidin zu 2-(4-Fluor-3-aminophenyl-imino)-imidazolidin.

Die Reduktion kann durch Hydrierung in Gegenwart von feinverteilten Metallkatalysatoren (z.B. Palladium, Platin, Raney-Nickel) bei Normal- oder Überdruck oder mittels nascierendem Wasserstoff (z.B. durch Hydrazin in Gegenwart von Raney-Nikkel) erfolgen.

Die erfindungsgemässen 2-Phenyl-iminoimidazolidine der Formel (I) können auf übliche Weise in ihre physiologisch verträglichen Säureadditionssalze überführt werden. Zur Salzbildung geeignete Säuren sind beispielsweise Salzsäure, Bromwasserstoffsäure, Jodwasserstoffsäure, Fluorwasserstoffsäure, Schwefelsäure, Phosphorsäure, Salpetersäure, Essigsäure, Propionsäure, Buttersäure, Capronsäure, Valeriansäure, Oxalsäure, Malonsäure, Bernsteinsäure, Maleinsäure, Fumarsäure, Milchsäure, Weinsäure, Zitronensäure, Äpfelsäure, Benzoesäure, p-Hydroxybenzoesäure, p-Aminobenzoesäure, Phthalsäure, Zimtsäure, Salicylsäure, Ascorbinsäure, Methansulfonsäure, Äthanphosphonsäure und 8-Chlortheophyllin.

Die neuen Verbindungen sowie deren Säureadditionssalze haben wertvolle therapeutische Eigenschaften. Durch pharmakologische Untersuchungen an Ratten und Katzen wurde festgestellt, dass sie eine praesynaptische Stimulierung der peripheren α-Adrenorezeptoren bewirken und dadurch eine periphere neurosympatische und -vagale Transmissionshemmung ausüben. Wegen des praktischen Fehlens zentraler Wirkungen ist eine Beeinflussung des Blutdrucks nicht zu beobachten. Die neuen Verbindungen können z.B. bei der Behandlung der Migräne Anwendung finden. Die Verbindungen der allgemeinen Formel (I) und deren Säureadditionssalze können enteral oder auch parenteral angewandt werden. Die Dosierung liegt bei 0,5 bis 50 mg, vorzugsweise bei 1 bis 10 mg bei oraler Applikation.

Die Verbindungen der Formel (I) bzw. ihre Säureadditionssalze können auch mit andersartigen Wirkstoffen zum Einsatz gelangen. Geeignete galenische Darreichungsformen sind beispielsweise Tabletten, Kapseln, Zäpfchen, Lösungen oder Pulver; hierbei können zu deren Herstellung die üblicherweise verwendeten galenischen Hilfs-, Träger-, Spreng- oder Schmiermittel oder Substanzen zur Erzielung einer Depotwirkung Anwendung finden.

Die folgenden Beispiele erläutern die Erfindung.

## Herstellungsbeispiele

### Beispiel 1

2-(3-Fluor-4-methylphenylimino)-imidazolidin

14,03 g (0,043 Mol) N-(3-Fluor-4-methylphenyl)-S-methylthiuroniumjodid werden zusammen mit 4,35 ml (~150%) Äthylendiamin in 58 ml Methanol 8 Stunden unter Rührung am Rückflusskühler erhitzt. Hierauf wird im Vakuum das Lösungsmittel abdestilliert und der Rückstand in 1n Salzsäure gelöst. Die salzsaure Lösung wird mit 5n KOH auf pH 7 gepuffert und 2mal mit Äther extrahiert (die Ätherextrakte werden verworfen) und anschliessend mit 50%iger Kalilauge alkalisiert. Es fällt dabei ein Öl aus, welches in kurzer Zeit durchkristallisiert. Nach dem Absaugen wird mit Wasser gewaschen und getrocknet.

Ausbeute: 6,1 g = 73,41% der Theorie
Schmp.: 109,5–110,5 °C
Rf: 0,1; System: Benzol 50, Dioxan 40, Äthanol 5, konz. $NH_4OH$ 5 (Volumenteile)
Träger: Kieselgel G + Leuchtpigment, Sichtbarmachung: UV und Kaliumjodplatinat

| | | | | |
|---|---|---|---|---|
| Ber.: | C 62,16 | H 6,26 | F 9,83 | N 21,75 |
| Gef.: | C 62,44 | H 6,44 | F 9,84 | N 21,04 |

### Beispiel 2

2-(4-Fluor-3-methylphenylimino)-imidazolidin

46 g (0,141 Mol) N-(4-Fluor-3-methylphenyl)-S-methylthiuroniumjodid werden zusammen mit 14,1 ml (~150%) Äthylendiamin in 190 ml Methanol 5 Stunden unter Rühren am Rückflusskühler erhitzt. Hierauf wird im Vakuum das Lösungsmittel abdestilliert und der Rückstand in 1n Salzsäure gelöst. Die salzsaure Lösung wird mit 5n KOH auf pH 7 gepuffert und 2mal mit Äther extrahiert (die Ätherextrakte werden verworfen). Nach Versetzen mit Aktivkohle und Filtration wird mit 50%iger KOH alkalisiert. Die Base fällt zunächst ölig aus, um nach Überschichtung mit Petroläther (40–80 °C) und gutem Rühren zu kristallisieren. Sie wird abgesaugt, mit Wasser gewaschen und getrocknet.

Ausbeute: 9,4 g = 34,50% der Theorie
Schmp.: 113–114 °C
Rf: 0,3; System: Benzol 50, Dioxan 40, Äthanol 5, konz. $NH_4OH$ 5 (jeweils Volumenteile)
Träger: Kieselgel G + Leuchtpigment, Sichtbarmachung: UV und Kaliumjodplatinat

| | | | | |
|---|---|---|---|---|
| Ber.: | C 62,16 | H 6,26 | F 9,83 | N 21,75 |
| Gef.: | C 62,04 | H 6,13 | F 9,85 | N 21,78 |

### Beispiel 3

2-(4-Fluor-3-nitrophenylimino)-imidazolidin

4,5 g (0,0173 Mol) 1-Acetyl-2-(4-fluor-3-nitrophenyl-imino)-imidazolidin, Fp. 150–151 °C, werden 10 Stunden in 66 ml Methanol unter Rühren am Rückflusskühler erhitzt. Nach dem Abkühlen wird vom Unlöslichen filtriert, das Lösungsmittel im Vakuum abdestilliert und der verbleibende Rückstand in 1n Salzsäure gelöst. Die salzsaure Lösung wird dann bei aufsteigenden pH-Werten (stufenweises Alkalisieren mit 2n Natronlauge) fraktioniert mit Äther extrahiert und ab pH 8,5 fraktioniert, gefällt und abgesaugt. Die festen Fraktionen werden vereint und getrocknet.

Ausbeute: 1,7 g = 43,81% der Theorie
Schmp.: 146–147 °C
Rf: 0,4; System: Benzol 50, Dioxan 40, Äthanol 5, konz. $NH_4OH$ 5 (jeweils Volumenteile)
Träger: Kieselgel G + Leuchtpigment, Detektor: UV, Kaliumjodplatinat.

| | | | | |
|---|---|---|---|---|
| Ber.: | C 48,21 | H 4,05 | F 8,48 | N 24,99 |
| Gef.: | C 47,55 | H 4,02 | F 8,71 | N 24,41 |

### Beispiel 4

2-(4-Fluor-3-chlorphenylimino)-imidazolidin

20,8 g (0,06 Mol) N-(3-Chlor-4-fluorphenyl)-S-methylthiuroniumjodid werden zusammen mit 6 ml (~150%) Äthylendiamin in 82 ml Methanol 4 Stunden unter Rühren am Rückflusskühler erhitzt. Hierauf wird im Vakuum das Lösungsmittel abdestilliert und der Rückstand in 1n Salzsäure gelöst. Die salzsaure Lösung wird mit 5n KOH auf pH 7 gepuffert und 2mal mit Äther extrahiert (die Ätherphasen werden verworfen) und anschliessend mit 50%iger KOH alkalisiert. Es fällt dabei ein Öl aus, welches in kurzer Zeit durchkristallisiert. Nach dem Absaugen wird mit Wasser gewaschen und getrocknet.

Ausbeute: 7,2 g = 56,17% der Theorie
Schmp.: 118,5–119,5 °C
Rf.: 0,5; System: Benzol 50, Dioxan 40, Äthanol 5, konz. $NH_4OH$ 5 (jeweils Volumenteile)
Träger: Kieselgel G + Leuchtpigment, Detektor: UV, Kaliumjodplatinat

| | | | | | |
|---|---|---|---|---|---|
| Ber.: | C 50,59 | H 4,25 | Cl 16,60 | F 8,89 | N 19,67 |
| Gef.: | C 49,80 | H 4,64 | Cl 16,57 | F 9,25 | N 19,42 |

### Beispiel 5

2-(4-Fluor-3-aminophenylimino)-imidazolidin

5,5 g (0,0225 Mol) 2-(4-Fluor-3-nitrophenyl-imino)-imidazolidin werden in 40 ml Methanol gelöst und unter Normaldruck bis zur theoretischen Wasserstoffaufnahme hydriert (Raney-Nickel). Hierauf wird durch Absaugen über Aktivkohle vom Raney-Nickel befreit und das Filtrat im Vakuum eingeengt. Zur Reinigung wird der Rückstand fraktioniert über eine 400-g-Kieselgelsäule eluiert (Elutionsmittelgemisch: Essigester 7, Isopropanol 3, konz. $NH_4OH$ 1, jeweils Volumenteile). Die dünnschichtchromatografisch einheitlichen Fraktionen werden vereinigt und im Vakuum bis zur Gewichtskonstanz eingeengt. Das verbleibende dikke Öl wird in wenig Methanol gelöst und mit ätherischer Salzsäure bis zur sauren Reaktion versetzt. Anschliessend wird mit Äther gefällt und das ausgefallene Salz abgesaugt, mit Äther gewaschen und getrocknet.

Ausbeute: 3,7 g = 61,56% der Theorie
Schmp.: 222 °C
Rf.: 0,08; System: Benzol 50, Dioxan 40, Äthanol 5, konz. $NH_4OH$ 5 (jeweils Volumenteile)
Träger: Kieselgel G + Leuchtpigment, Detektor: UV, Kaliumjodplatinat

| | | | | | |
|---|---|---|---|---|---|
| Ber.: | C 40,46 | H 4,90 | Cl 26,55 | F 7,11 | N 20,57 |
| Gef.: | C 39,53 | H 5,18 | Cl 26,26 | F 6,58 | N 20,37 |

Formulierungsbeispiele

Beispiele 6: Tabletten

| | |
|---|---|
| Wirkstoff gemäss Erfindung | 5 mg |
| Maisstärke | 160 mg |
| sek. Calciumphosphat | 250 mg |
| Magnesiumstearat | 5 mg |
| insgesamt | 420 mg |

Herstellung:
Die einzelnen Bestandteile werden intensiv miteinander vermischt und die Mischung in üblicher Weise granuliert. Das Granulat wird zu Tabletten von 420 mg Gewicht verpresst, von denen jede 5 mg Wirkstoff enthält.

Beispiel 7: Gelatinekapseln

Der Inhalt der Kapseln setzt sich wie folgt zusammen:

| | |
|---|---|
| Wirkstoff gemäss Erfindung | 3 mg |
| Maisstärke | 172 mg |
| insgesamt | 175 mg |

Herstellung:
Die Bestandteile des Kapselinhaltes werden intensiv vermischt und 175-mg-Portionen der Mischung in Gelatinekapseln geeigneter Grösse abgefüllt. Jede Kapsel enthält 3 mg des Wirkstoffs.

Beispiel 8: Injektionslösungen

Die Lösung wird aus folgenden Bestandteilen hergestellt:

| | |
|---|---|
| Wirkstoff gemäss Erfindung | 1,0 Gewichtsteile |
| Natriumsalz der Äthylendiamintetraessigsäure | 0,2 Gewichtsteile |
| destilliertes Wasser ad | 1000,0 Gewichtsteile |

Herstellung:
Der Wirkstoff und das Natriumsalz der Äthylendiamintetraessigsäure werden in genügend Wasser gelöst und mit Wasser auf das gewünschte Volumen aufgefüllt. Die Lösung wird von suspendierten Partikeln filtriert und in 2-ml-Ampullen unter aseptischen Bedingungen abgefüllt. Zuletzt werden die Ampullen sterilisiert und verschlossen. Jede Ampulle enthält 2 mg Wirkstoff.

Beispiel 9: Tropfen

| | |
|---|---|
| Wirkstoff gemäss Erfindung | 0,20 g |
| p-Hydroxybenzoesäuremethylester | 0,07 g |
| p-Hydroxybenzoesäurepropylester | 0,03 g |
| entmineralisiertes Wasser ad | 100 ml |

**Patentansprüche für die Vertragsstaaten: BE, CH, DE, FR, GB, IT, LU, NL, SE**

1. 3,4-disubstituierte 2-Phenylimino-imidazolidine der allgemeinen Formel (I)

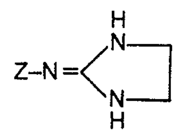 (I)

in der Z einen Rest aus der Gruppe 3-Fluor-4-methylphenyl, 4-Fluor-3-methylphenyl, 4-Fluor-3-nitrophenyl, 4-Fluor-3-chlorphenyl, 4-Fluor-3-aminophenyl bedeutet sowie deren Säureadditionssalze.

2. Verfahren zur Herstellung von Verbindungen nach Anspruch 1, dadurch gekennzeichnet, dass man

a) eine Verbindung der Formel (II)

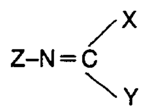 (II)

in der Z die oben genannte Bedeutung besitzt und X und Y, die gleich oder verschieden sein können, ein Chloratom, eine Alkylthiogruppe mit 1 bis 4 Kohlenstoffatomen, eine Amino- oder Ammoniumgruppe bedeuten, mit Äthylendiamin oder dessen Säureadditionssalzen umsetzt; oder

b) aus einem 1-Acyl-2-phenylamino-imidazolin der allgemeinen Formel (III)

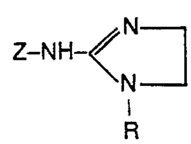 (III)

in der Z die oben genannte Bedeutung besitzt und R einen aliphatischen Acylrest bedeutet, den aliphatischen Acylrest hydrolytisch abspaltet; oder

c) zwecks Herstellung von 2-(4-Fluor-3-aminophenylimino)-imidazolidin 2-(4-Fluor-3-nitrophenylimino)-imidazolin hydriert und gegebenenfalls die so erhaltene Verbindung in ein physiologisch unbedenkliches Säureadditionssalz überführt.

3. Pharmazeutische Zubereitungen, dadurch gekennzeichnet, dass sie als Wirkstoff eine oder mehrere Verbindungen der allgemeinen Formel (I) oder deren physiologisch verträgliche Säureadditionssalze nach Anspruch 1 enthalten.

4. Verfahren zur Herstellung von Arzneimitteln nach Anspruch 3, dadurch gekennzeichnet, dass man eine oder mehrere Verbindungen nach Anspruch 1 mit üblichen galenischen Hilfs-, Träger-, Spreng- oder Schmiermitteln oder Substanzen zur Erzielung einer Depotwirkung zu Tabletten, Kapseln, Zäpfchen, Lösungen oder Pulvern formuliert.

5. Verbindungen nach Anspruch 1 zur Verwendung bei der Behandlung der Migräne.

**Patentansprüche für den Vertragsstaat: AT**

1. Verfahren zur Herstellung von 3,4-disubstituierten 2-Phenylimino-imidazolidinen der allgemeinen Formel (I)

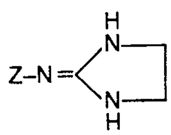
(I)

in der Z einen Rest aus der Gruppe 3-Fluor-4-methylphenyl, 4-Fluor-3-methylphenyl, 4-Fluor-3-nitrophenyl, 4-Fluor-3-chlorphenyl, 4-Fluor-3-aminophenyl bedeutet sowie von deren Säureadditionssalzen, dadurch gekennzeichnet, dass man

a) eine Verbindung der Formel (II)

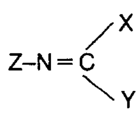
(II)

in der Z die oben genannte Bedeutung besitzt und X und Y, die gleich oder verschieden sein können, ein Chloratom, eine Alkylthiogruppe mit 1 bis 4 Kohlenstoffatomen, eine Amino- oder Ammoniumgruppe bedeuten, mit Äthylendiamin oder dessen Säureadditionssalzen umsetzt; oder

b) aus einem 1-Acyl-2-phenylamino-imidazolin der allgemeinen Formel (III)

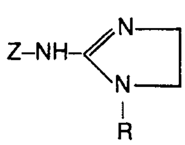
(III)

in der Z die oben genannte Bedeutung besitzt und R einen aliphatischen Acylrest bedeutet, den aliphatischen Acylrest hydrolytisch abspaltet; oder

c) zwecks Herstellung von 2-(4-Fluor-3-amino-phenylimino-imidazolidin 2-(4-Fluor-3-nitrophenylimino)-imidazolin hydriert und gegebenenfalls die so erhaltene Verbindung in ein physiologisch unbedenkliches Säureadditionssalz überführt.

2. Verfahren zur Herstellung von Arzneimitteln, dadurch gekennzeichnet, dass man eine oder mehrere Verbindungen nach Anspruch 1 mit üblichen galenischen Hilfs-, Träger-, Spreng- oder Schmiermitteln oder Substanzen zur Erzielung einer Depotwirkung zu Tabletten, Kapseln, Zäpfchen, Lösungen oder Pulvern formuliert.

**Claims for the contracting states: BE, CH, DE, FR, GB, IT, LU, NL, SE**

1. 3,4-Disubstituted 2-phenylimino-imidazolidines of general formula (I)

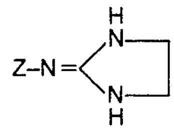
(I)

wherein Z represents a radical selected from the group comprising 3-fluoro-4-methylphenyl, 4-fluoro-3-methylphenyl, 4-fluoro-3-nitrophenyl, 4-fluoro-3-chlorophenyl and 4-fluoro-3-aminophenyl, and the acid addition salts thereof.

2. Process for the preparation of compounds according to claim 1, characterised in that

a) a compound of formula (II)

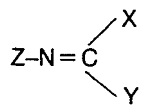
(II)

wherein Z is as hereinbefore defined and X and Y, which may be the same or different, represent a chlorine atom, an akylthio group with 1 to 4 carbon atoms or an amino or ammonium group, is reacted with ethylenediamine or an acid addition salt thereof; or

b) the aliphatic acyl group is hydrolytically split off from a 1-acyl-2-phenylamino-imidazoline of general formula (III)

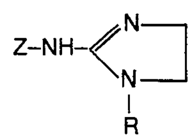
(III)

wherein Z is as hereinbefore defined and R represents an aliphatic acyl group; or

c) in order to prepare 2-(4-fluoro-3-amino-phenylimino)-imidazolidine, 2-(4-fluoro-3-nitro-phenylimino)-imidazoline is hydrogenated and, if desired, the compound thus obtained is converted into a physiologically acceptable acid addition salt.

3. Pharmaceutical preparations, characterised in that they contain, as active substance, one or more compounds of general formula (I) or the physiologically acceptable acid addition salts thereof according to claim 1.

4. Process for the preparation of pharmaceutical compositions according to claim 3, characterised in that one or more compounds as claimed in claim 1 are processed with conventional galenic excipients, carriers, disintegrants or lubricants or substances for obtaining delayed release, to form tablets, capsules, suppositories, solutions or powders.

5. Compounds according to claim 1 for use in the treatment of migraine.

**Claims for the contracting state: AT**

1. Process for the preparation of 3,4-disubstituted 2-phenylimino-imidazolidines of general formula (I)

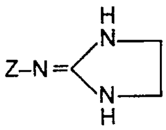 (I)

wherein Z represents a radical selected from the group comprising 3-fluoro-4-methylphenyl, 4-fluoro-3-methylphenyl, 4-fluoro-3-nitrophenyl, 4-fluoro-3-chlorophenyl and 4-fluoro-3-aminophenyl, and the acid additions salts thereof, characterised in that

a) a compound of formula (II)

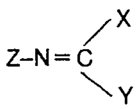 (II)

wherein Z is as hereinbefore defined and X and Y, which may be the same or different, represent a chlorine atom, an alkylthio group with 1 to 4 carbon atoms or an amino or ammonium group, is reacted with ethylenediamine or an acid addition salt thereof; or

b) the aliphatic acyl group is hydrolytically split off from a 1-acyl-2-phenylamino-imidazoline of general formula (III)

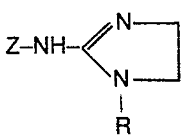 (III)

wherein Z is as hereinbefore defined and R represents an aliphatic acyl group; or

c) in order to prepare 2-(4-fluoro-3-aminophenylimino)-imidazolidine, 2-(4-fluoro-3-nitrophenylimino)-imidazoline is hydrogenated and, if desired, the compound thus obtained is converted into a physiologically acceptable acid addition salt.

2. Process for the preparation of pharmaceutical compositions, characterised in that one or more compounds according to claim 1 are processed with conventional galenic excipients, carriers, disintegrants or lubricants or substances for obtaining delayed release, to form tablets, capsules, suppositories, solutions or powders.

**Revendications pour les états contractants: BE, CH, DE, FR, GB, IT, LU, NL, SE**

1. 2-phénylimino-imidazolidines 3,4-disubstituées de formule générale (I)

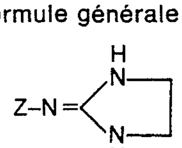 (I)

dans laquelle Z représente un radical choisi dans le groupe 3-fluoro-4-méthylphényle, 4-fluoro-3-méthylphényle, 4-fluoro-3-nitrophényle, 4-fluoro-3-chlorophényle, 4-fluoro-3-aminophényle ainsi que leurs sels d'addition avec des acides.

2. Procédé pour la préparation de composés selon la revendication 1, caractérisé en ce que

a) on fait réagir un composé de formule (II)

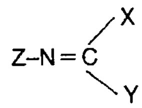 (II)

dans laquelle Z possède la signification mentionnée plus haut et X et Y, qui peuvent être identiques ou différents, représentent un atome de chlore, un groupe alcoylthio avec 1 à 4 atomes de carbone, un groupe amino ou ammonium, avec l'éthylènediamine ou ses sels d'addition avec des acides, ou

b) à partir d'une 1-acyl-2-phénylamino-imidazoline de formule générale (III)

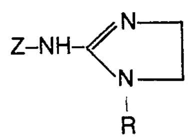 (III)

dans laquelle Z possède la signification mentionnée plus haut et R représente un radical acyle aliphatique, on clive hydrolytiquement le radical acyle aliphatique; ou

c) dans le but de préparer la 2-(4-fluoro-3-aminophénylimino)-imidazolidine on hydrogène la 2-(4-fluoro-3-nitrophénylimino)-imidazoline et on transforme éventuellement le composé ainsi obtenu en un sel d'addition d'acide physiologiquement inoffensif.

3. Préparations pharmaceutiques, caractérisées en ce qu'elles contiennent en tant que substance active un ou plusieurs composés de formule générale (I) ou leurs sels d'addition d'acides physiologiquement supportables selon la revendication 1.

4. Procédé pour la préparation de médicaments selon la revendication 3, caractérisè en ce qu'on formule un ou plusieurs composés selon la revendication 1 avec des adjuvants, excipients, désagrégeants ou lubrifiants ou substances pour obtenir un effet retard habituels en comprimés, capsules, suppositoires, solutions ou poudres.

5. Composés selon la revendication 1 pour l'utilisation dans le traitement de la migraine.

**Revendications pour l'état contractant: AT**

1. Procédé pour la préparation de 2-phénylimino-imidazolidines 3,4-disubstituées de formule générale (I)

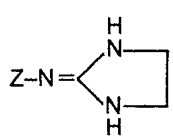 (I)

dans laquelle Z représente un radical choisi dans le groupe 3-fluoro-4-méthylphènyle, 4-fluoro-3-méthylphényle, 4-fluoro-3-nitrophényle, 4-fluoro-3-chlorophényle, 4-fluoro-3-aminophényle, ainsi

que de leurs sels d'addition avec des acides, caractérisé en ce que

a) on fait réagir un composé de formule (II)

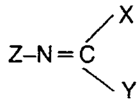

(II)

dans laquelle Z possède la signification mentionnée plus haut et X et Y, qui peuvent être identiques ou différents, représentent un atome de chlore, un groupe alcoylthio avec 1 à 4 atomes de carbone, un groupe amino ou ammonium, avec l'éthylènediamine ou ses sels d'addition d'acides; ou

b) à partir d'une 1-acyl-2-phénylamino-imidazoline de formule générale (III)

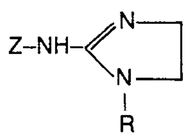

(III)

dans laquelle Z possède la signification mentionnée plus haut et R représente un radical acyle aliphatique, on clive hydrolytiquement le radical acyle aliphatique; ou

c) dans le but de préparer la 2-(4-fluoro-3-aminophénylimino)-imidazolidine, on hydrogène la 2-(4-fluoro-3-nitrophénylimino)-imidazoline et on transforme éventuellement le composé ainsi obtenu en un sel d'addition d'acide physiologiquement inoffensif.

2. Procédé pour la préparation de médicaments, caractérisé en ce qu'on formule un ou plusieurs composés selon la revendication 1 avec des adjuvants, excipients, désagrégeants ou lubrifiants ou substances pour obtenir un effet retard habituels en comprimés, capsules, suppositoires, solutions ou poudres.